Europäisches Patentamt

European Patent Office

Office européen des brevets

(11) Numéro de publication : **0 511 895 A1**

# (12) DEMANDE DE BREVET EUROPEEN

(21) Numéro de dépôt : 92401107.5

(22) Date de dépôt : 21.04.92

(51) Int. Cl.$^5$ : **A61K 33/34,** A61K 33/32, A61K 33/30, A61K 33/24, A61K 33/18, A61K 33/16, A61K 33/06, A61K 33/04, A61K 33/00, A61K 31/72, A61K 31/595

(30) Priorité : 30.04.91 FR 9105286

(43) Date de publication de la demande :
04.11.92 Bulletin 92/45

(84) Etats contractants désignés :
CH DE LI

(71) Demandeur : **Baritiu, Georges**
**11, Rue Bisson**
**F-75020 Paris (FR)**

(72) Inventeur : **Baritiu, Georges**
**11, Rue Bisson**
**F-75020 Paris (FR)**
Inventeur : **Ciustea, Gheorghe**
**Strada Soldat Zambila Ionita, Sect. II 2e étage**
**73500 Bucarest (RO)**

(54) **Compositions sanogènes diététiques complexes.**

(57)   La présente invention a pour objet la réalisation de nouvelles compositions sanogènes diététiques complexes et originales, possédant de multiples actions biologiques.
   Leurs principales caractéristiques étant : une action énergisante et réconfortante, une action de stimulation immunitaire, une action anti-alcoolique, (neuro-hépato-protectrice), une action anti-mutagène, une action de stimulation de l'appétit sexuel, une action gériatrique, une action infarcto-préventive, enfin et surtout, une action anticancéreuse.
   Ces compositions sont destinées à une très large consommation publique, pouvant être présentées et commercialisées sous des formes, de préparatios diététiques, les plus diverses.

EP 0 511 895 A1

La présente proposition d'invention se donne comme objectif un certain type de compositions complexes réalisées à partir de produits possédant des multiples actions complémentaires et synergiques : action énergisante, action réconfortante, action de stimulation immunitaire, action antialcoolique (neuro-hépato-protectrice), action anti-mutagène, action de stimulation de l'appétit sexuel, action infarcto-préventive, mais surtout une action anti-cancéreuse.

Certains vins toniques préparés, par exemple, en pharmacie comme aussi des jus de fruits ou des extraits de plantes naturelles contenant des vitamines ou des oligo-éléments essayent de répondre aussi à la nécessité de l'obtention de certaines préparations dotées d'une réelle action sanogène.

La présente proposition d'invention a comme but le perfectionnement de toutes ces préparations connues jusqu'à nos jours, par l'utilisation de certains composants nouveaux possédant une activité sanogène plus étendue et beaucoup plus efficace, tout particulièrement par leur action anticancéreuse préventive et même curative, dans le cas de néoplasies naissantes.

Ces composants n'agissent pas seulement d'une manière complémentaire, mais aussi synergique. Ces compositions sanogènes complexes peuvent être administrées le plus convenablement sous forme de certaines préparations diététiques les plus diverses : boissons, crèmes, bonbons, biscuits,des sticks, des yaourts, du chocolat blanc, des comprimés effervescents, donc des moyens excellents et agréables de faciliter un large accès à une consommation généralisée.

Ainsi donc, nous ne connaissons pas, à ce jour, l'existence d'une boisson analcoolique, aux qualités organoleptiques aussi agréables dotée, en même temps, de qualités sanogènes déjà mentionnées, possédant en tout premier lieu une activité anti-cancéreuse multiple et d'une indéniable intensité.

Il est notoirement connu le phénomène que les humains sont constamment exposés à l'action de certains agents physiques, mutagènes, gérontogènes et cancérigènes et aussi que ces effets s'amplifient progressivement, au fur et à mesure, de l'évolution de la société humaine.

Par conséquent, nous considérons particulièrement utile de tenter, de tout mettre en oeuvre, afin de combattre ces effets négatifs par une consommation régulière de certaines préparations diététiques, à partir de ces compositions sanogènes complexes qui font l'objet de la présente proposition d'invention.

Ces compositions sont caractérisées principalement par la présence dans leur constitution des composants suivants :

1. par une certaine proportion de produits énergisants et réconfortants;

2. par une certaine proportion de stimulateurs immunitaires non spécifiques;

3. par une proportion de certains produits qui empêchent les mutations cellulaires;

4. par une proportion de certains produits qui empêchent la multiplication des cellules ayant subi des mutations leur évitant ainsi l'évolution vers des clones et des tumeurs;

5. par une proportion de certains produits qui détermine la réversion (la différenciation) des cellules cancéreuses en cellules normales (les réverseurs);

6. par une proportion de certains produits qui assurent certaines qualités organoleptiques.

Pour chacun de ces six groupes de composants mentionnés, nous avons choisi les prototypes les plus représentatifs et les plus adaptés chez lesquels les effets adverses éventuels ne peuvent se manifester qu'en cas d'une consommation plusieurs fois supérieure aux quantités consommées habituellement.

La cosommation quotidienne recommandée est approximativement de 100 ml à 100 ml par jour pendant cinq jours de la semaine en tenant compte, bien sûr, des nécessités et du poids du consommateur. Pour des personnes exposées tout particulièrement à des noxes mutagènes et cancérigènes, l'on peut doubler ou même tripler la concentration des préparations, en substances actives (préparations "FORTE").

Nous précisons également que pour le maintien de la boisson (de la préparation), en état de conservation (aseptique), cela se réalise par la pasteurisation ou par le passage à travers des filtres dont le diamètre des pores ne dépasse pas 1 - 1,5 microns.

Afin d'influencer, très positivement, les propriétés organoleptiques des préparations diététiques, certains composants sont utilisés sous forme d'estères poli-alcooliques ou poli-carboxyliques originaux. On a tenu compte, en même temps de la compatibilité chimique et biologique des composants.

Des recherches expérimentales ont été effectuées sur des animaux et ultérieurement aussi sur l'homme qui ont confirmé, indubitablement, les effets sanogènes multiples et anti-cancéreux des compositions proposées. Certains composants, comme par exemple, la CHOLINE, la METHIONINE, l'acide ASCORBIQUE et le MOLIBDENE sont utilisés, avant tout, pour leur contribution à l'effet anti-cancéreux, mais en même temps, pour d'autre effets bénéfiques. Ils assurent, simultanément, une protection partielle de l'organisme humain, en cas de consommation d'alcool, en vertu de certains mécanismes biochimiques connus (la neutralisation, en bonne partie, de l'ACETATEALDEHIDE, produit issu de la métabolisation de l'alcool dans l'organisme humain).

Nous donnons en continuation un exemple de composition sanogène complexe pour 1 000 ml

```
 1 - ACIDE ASCORBIQUE                                      1000 mg
 2 - RETINOL CX                                              80 mg
 3 - ACIDE RETINOIQUE                                         8 mg
 4 - B CAROTENE                                              20 mg
 5 - ALPHA TOCOPHEROL (X)                                   300 mg
 6 - VITAMINE F sous forme de mono-ester de Sorbitol        300 mg
 7 - 125 DIHYDROXY-VITAMINE D3                             0,005 mg
 8 - CHOLINE                                                180 mg
 9 - METHIONINE                                             140 mg
10 - SARCOSINE                                                4 mg
11 - ACIDE FOLIQUE                                           10 mg
12 - CYSTEYNE (X)                                            40 mg
13 - ACIDE ELLAGIQUE                                         10 mg
14 - TANNIN DE THEASINENSIS                                  20 mg
15 - ROTINE                                                  40 mg
16 - ANTHOLYANOSINES VEGETALES                               60 mg
17 - SELENIUM-METHIONINE                                      2 mg
18 - MOLIBDATE DE SODIUM                                      1 mg
19 - VANADATE DE POTASSIUM                                  0,5 mg
20 - CARBONATE DE CALCIUM                                    40 mg
21 - CARBONATE DE MAGNESIUM                                  60 mg
22 - OXYDE DE ZINC                                           10 mg
23 - GLUCONATE DE MANGANESE                                  25 mg
24 - GLUCONATE DE CUIVRE                                     10 mg
25 - CHROME-PROTEINES COMPLEXE(ou PROTEINATE DE CHROME)       2 mg
26 - FLUORURE DE POTASSIUM                                    6 mg
27 - IODURE DE POTASSIUM                                    1,5 mg
28 - AGENT EDULCORANT (ASPARTAM) et AROMES               47.000 mg
29 - BIOXYDE DE CARBON                                    3.000 mg
30 - EAU POTABLE DE QUALITE EXTRA                           950 ml
```

Les substances marquées par un (X), nous les utilisons dans la composotion de certaines préparations diététiques sous forme de mono-estères de certains poli-alcools et poli-acides physiologiques en vue d'assurer leur solubilité dans l'eau ou l'amélioration de certaines propriétés organoleptique.

Des compositions similaires qui contiennent des substances de la même classe (groupe) ou qui possèdent les mêmes actions sanogènes, visant les mêmes buts, tombent sous l'incidence de ce brevet.

Tableau des composants contenus dans nos compositions sanogènes avec leurs valeurs quantitatives (doses) autorisées par Kg/corps quotidiennement.

| | consommation quotidienne habituelle | | limites normales recommandée | |
|---|---|---|---|---|
| -ACIDE ASCORBIQUE | 9,57 | mg | 142,85 | mg |
| -RETINOL | 0,28 | mg | 3,00 | mg |
| -ACIDE RETINOÏQUE | 0,028 | mg | 0,85 | mg |
| -B CAROTENE | 0,00714 | mg | 0,43 | mg |
| -ALPHATOCOPHEROL | 1,071 | mg | 34,28 | mg |
| -VITAMINE F | 1,071 | mg | 25,00 | mg |
| -1.25 DIHYDROXY-VITAMINE D5 | 0,0000178 | mg | 0,0080 | mg |
| -CHOLINE | 0,6428 | mg | 7,00 | mg |
| -METHIONINE | 0,5 | mg | 71,42 | mg |
| -SARCOSINE | 0,01428 | mg | 1,20 | mg |
| -ACIDE FOLIQUE | 0,0357 | mg | 0,72 | mg |
| -CYSTEINE | 0,1428 | mg | 2,88 | mg |
| -ACIDE ELLARGIQUE | 0,03571 | mg | 1,60 | mg |
| -TANNIN | 0,07142 | mg | 1,15 | mg |
| -RUTINE | 0,1428 | mg | 2,85 | mg |
| -ANTHOCYANOSINES | 0,2142 | mg | 3,00 | mg |
| -SELENIUM/sous forme de composés compatibles avec l'acide ascorbique | 0,007142 | mg | 0,15 | mg |
| -MOLIBDATE DE SODIUM | 0,00357 | mg | 0,15 | mg |
| -VANADATE DE POTASSIUM | 0,001785 | mg | 0,05 | mg |
| -CARBONATE DE CALCIUM | 0,1428 | mg | 70,00 | mg |
| -CARBONATE DE MAGNESIUM | 0,2142 | mg | 52,00 | mg |
| -OXYDE DE ZINC | 0,0357 | mg | 0,43 | mg |
| -GLUCONATE DE MANGANESE | 0,08928 | mg | 1,07 | mg |
| -GLUCONATE DE CUIVRE | 0,03571 | mg | 0,85 | mg |
| -PROTEINATE DE CHROME | 0,007142 | mg | 0,16 | mg |
| -FLUORURE DE POTASSIUM | 0,02142 | mg | 0,37 | mg |
| -IODURE DE POTASSIUM | 0,005357 | mg | 0,35 | mg |

Plusieurs substances actives biologiquement qui entrent dans les compositions sanogènes proposées, l'on peut les trouver aussi dans certaines plantes ce qui permettrait que ces compositions puissent être réalisées aussi à partir d'extraits de plantes auxquels l'on peut ajouter les composants qui seraient manquants ou en qualité insuffisante.

Les quantités (les doses) mentionnées plus haut peuvent être considérées, en diverses variantes, sans pour autant s'éloigner du cadre du brevet, à condition, bien sûr, de respecter, certaines limites acceptables biologiquement.

**Revendications**

1. Compositions sanogènes diététiques complexes constituées de substances, aux effets biologiques multiples:
   énergisantes, réconfortantes, immuno-stimulatrices, anti-mutagènes, anti-cancéreuse, gériatriques, stimulatrices de l'appétit sexuel, infarcto-préventives, anti-alcooliques, contenant dans leur composition:
   acide ascorbique, rétinol, acide rétinoique, beta-carotène, alpha-tocophérol, vitamine D3, 1.25 dihydroxi-vitamine D3, vitamine F, rutine, antocyanosides, acide ellagique, tannin, choline,methionine, sarcosine, acide folique, cysteine des dérivés bio-assimilables anorganiques ou organiques de zinc, de magnésium, de calcium, de molibdène, de selenium, de chrome, de cuivre, de manganèse, de vanadium, de fluor, de iode, comme aussi d'autres composants similaires ayant les mêmes actions sanogènes (certaines vitamines, certaines substances organique et certains oligo-élements).

2. Compositions sanogènes diététiques complexes selon la revendication 1, caractérisées en ce qu'elles possèdent une importante action énergisante, réconfortante et anti-infarctique ainsi que de stimulation dans certaines fonctions biologiques, réparatrices, immunitaires, hormonales, gériatriques et sexuelles. Les variations des quantités autorisées des substances bio-actives utilisées dans nos compositions par Kg/corps/jour sont les suivantes: Acide Ascorbique 0,02 - 200 mg, Retinol 0,01 - 25 mg, Acide Rétinoïque 0,01 - 10 mg, Beta Carotene 0,01 - 2,50 mg, Alpha-Tocophèrol 0,01 - 30 mg, Vitamine D3 100 - 60.000 U.I. , Vitamine F 0,01 - 50 mg, Methionine 0,005 - 70 mg, Anthocyanosimes 0,003 - 70 mg, Acide Folique 0,003 - 1,50 mg, Cysteine 0,002 - 3 mg, Magnesium 0,001 - 53 mg, calcium 0,01 - 80 mg, Molibdène 0,002 - 4 mg, Zinc 0,002 - 0,3 mg, Manganèse 0,001 - 2 mg, Vanadium 0,001 - 0,05 mg, chrome 0,001 - 0,180 mg, cuivre 0,002 - 0,850 mg, Selenium (sous forme de composés compatibles avec l'acide ascorbique) 0,001 - 0,15 mg, Fluor 0,001 - 0,35 mg, Iode 0,002 - 0,30 mg.

3. compositions sanogènes diététiques complexes selon les revendications 1 ou 2 caractérisées en ce que l'action ajoute des produits comme 1.25 DIHYDROXYVITAMINE D3 0,OOOO178 - 0,0080 mg par Kg-/corps/jour, SARCOSINE 0,001 - 1,20 mg Kg/corps/jour et TANNIN 0,001 - 1,4 mg Kg/corps/jour amplifient sensiblement l'action anti-cancéreuse des composants déjà mentionnés à la revendication 2, par un apport supplémentaire d'inhibition des mutations vers des clones et des tumeurs ainsi que par une action de différenciation (maturation) des cellules cancéreuses préexistantes (reverseurs).

4. Compositions sanogènes diététiques complexes selon les revendications 1,2 ou 3 caractérisées en ce que la METHIONINE, la CHOOLINE et la MOLIBDENE determinent une protection hépatique, cellulaire et biochimique importante vis-à-vis de l'action nocive de l'alcool éthylique.

5. Compositions sanogènes diététiques complexes selon les revendications 1,2,3 ou 4 caractérisées en ce que certaines substances actives sont utilisées sous la forme de structures chimiques (spécialement des estères-alcooliques ou policarboxyliques) qui assurent des qualités organoleptiques améliorées ainsi qu'un important espace de sécurité entre les doses actives et celles où peuvent apparaître des effets adverses.

6. Compositions sanogènes diététiques complexes selon les revendications 1,2,3,4 ou 5 cractérisées en ce qu'elles utilisent les agents d'édulcoration les moins nocifs, comme par exemple le glucose, l'aspartam dans des quantités tout à fait adéquates pour assurer des propriétés organoleptiques des plus agréables.

7. Compositions sanogènes diététiques complexes selon les revendications 1,2,3,4,5 ou 6 caractérisées en ce qu'elles utilisent pour certaines préparations diététique, comme par exemple, pour les boissons, du bioxyde de carbone alimentaire jusqu'à la saturation.

8. Compositions sanogènes diététiques complexes selon les revendications 1,2,3,4,5,6 ou 7 caractérisées en ce qu'elles se prêtent à être présentées et utilisées sous les formes diététiques les plus diverses: liquides, semi-solides, solides, comme par exemple, les boissons moléculaires ou colloïdales, des crèmes, des bonbons, des biscuits, des sticks, du chocolat blanc, des comprimés effervescents.

**Office européen des brevets**

# RAPPORT DE RECHERCHE EUROPEENNE

Numero de la demande

EP 92 40 1107

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 725 427 (ALBION INTERNATIONA, INC) Février 1988 <br> * revendication 1 * <br> --- | 1-8 | A 61 K 33/34 <br> A 61 K 33/32 <br> A 61 K 33/30 <br> A 61 K 33/24 <br> A 61 K 33/18 <br> A 61 K 33/16 <br> A 61 K 33/06 <br> A 61 K 33/04 <br> A 61 K 33/00 <br> A 61 K 31/72 <br> A 61 K 31/595 |
| A | GB-A-2 212 722 (THERAVIT LIMITED) <br> * revendications * <br> --- | 1-8 | |
| A | DE-A-3 800 968 (ASCHE, KARL) <br> * revendication 1 * <br> --- | 1-8 | |
| A | DE-A-3 800 969 (ASCHE, KARL) <br> * revendications * <br> --- | 1-8 | |
| A | EP-A-0 247 616 (RORER INTERNATIONAL) 1987 <br> * revendications * <br> ----- | 1-8 | |

**DOMAINES TECHNIQUES RECHERCHES (Int. Cl.5)**

A 61 K

Le présent rapport a été établi pour toutes les revendications

| Lieu de la recherche | Date d'achèvement de la recherche | Examinateur |
|---|---|---|
| LA HAYE | 03-07-1992 | LEHERTE C.F.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................
& : membre de la même famille, document correspondant

EPO FORM 1503 03.82 (P0402)

6